(19) Europäisches Patentamt
European Patent Office
Office européen des brevets

(11) EP 0 728 804 B1

(12) EUROPEAN PATENT SPECIFICATION

(45) Date of publication and mention of the grant of the patent: **08.09.1999 Bulletin 1999/36**

(51) Int Cl.[6]: **C08K 5/00**, C08K 3/36, C08L 29/04, C11D 17/00, A61L 9/01

(21) Application number: **95308399.5**

(22) Date of filing: **23.11.1995**

# (54) Extruded fragrance-containing polyvinyl alcohol and use thereof

Extrudierter, Parfüm-enthaltender Polyvinylalkohol und seine Verwendung

Alcool polyvinylique extrudé contenant du parfum et son utilisation

(84) Designated Contracting States:
**BE DE ES FR IE IT NL**

(30) Priority: **24.02.1995 US 394614**
**11.05.1995 GB 9509593**
**17.10.1995 NL 1001434**

(43) Date of publication of application:
**28.08.1996 Bulletin 1996/35**

(73) Proprietor: **INTERNATIONAL FLAVORS & FRAGRANCES INC.**
**New York New York 10019 (US)**

(72) Inventors:
- **McDermott, Keith J.**
  **Bound Brook, New Jersey 08805 (US)**
- **Teffenhart, John M.**
  **Edison, New Jersey 08817 (US)**
- **Shefer, Shmuel David**
  **East Brunswick, New Jersey 08816 (US)**
- **Greene, David A.**
  **Wanamassa, New Jersey 07712 (US)**
- **Smith, Leslie C.**
  **Jamesburg, New Jersey 08831 (US)**
- **Beck, Charles E.J.**
  **Summit, New Jersey 07901 (US)**

(74) Representative: **Brown, John David et al**
**FORRESTER & BOEHMERT**
**Franz-Joseph-Strasse 38**
**80801 München (DE)**

(56) References cited:
**WO-A-92/03532**
**US-A- 4 229 410**
**US-A- 4 339 356**

Remarks:
The file contains technical information submitted after the application was filed and not included in this specification

EP 0 728 804 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

The present invention relates to a composition of matter consisting of:

(a) extruded polyvinyl alcohol or partially hydrolyzed polyvinyl acetate with the maximum mole ratio of the acetyl moiety:hydroxyl moiety being about 3:1, having a number average molecular weight of from about 15,000 up to about 68,000 containing from 0 up to about 15% by weight of an added polar plasticizer;

(b) from about 1 up to about 20% by weight of a compatible fragrance contained within the polyvinyl alcohol or the partially hydrolyzed polyvinyl acetate;

(c) from 0 up to about 20% by weight of a "foaming agent" which is a first surfactant and which can be a detergent, contained within the polyvinyl alcohol or partially hydrolyzed polyvinyl acetate;

(d) from 0 up to about 20% by weight of hydrophobic silica contained within the polyvinyl alcohol or the partially hydrolyzed polyvinyl acetate;

(e) from 0 up to about 20% by weight of at least one second surfactant in addition to the aforementioned "foaming agent";

(f) from 0 up to about 5% of a water-soluble dye; and

(g) from 0 up to about 4% of a "foam booster".

The invention also contemplates toilet rim blocks having the following dimension ranges:

6.35 - 19.05 mm (1/4"-3/4") thickness;
6.35 - 101.6 mm (1/4"-4") width; and
6.35 - 127 mm (1/4"-5") length.

WO-A-92/03532 (Jeyes Limited) discloses a process for the production of lavatory cleaning blocks by extruding a mixture of ingredients, the mixture to be extruded comprising, based on the total weight of the mixture, (i) from 5 to 85% by weight of one or more surface active agents and (ii) from 0.5 to 40% by weight of one or more rheological control agents.

According to the present invention, there is provided a composition of matter consisting of:

(a) polyvinyl alcohol or partially hydrolyzed polyvinyl acetate with a maximum mole ratio of acetyl moiety:hydroxyl moiety being 3:1, having a number average molecular weight of from 15,000 up to 68,000 containing from 0 up to 15% by weight of an added polar plasticizer;
(b) from 1 up to 20% by weight of compatible fragrance contained within the polyvinyl alcohol or partially hydrolyzed polyvinyl acetate;
(c) from 0 up to 20% by weight of foaming agent contained within the polyvinyl alcohol or partially hydrolyzed polyvinyl acetate;
(d) from 0 up to 20% by weight of hydrophobic silica contained within the polyvinyl alcohol or partially hydrolyzed polyvinyl acetate;
(e) from 0 up to 20% by weight of at least one surfactant in addition to the said foaming agent;
(f) from 0 up to 5% by weight of water-soluble dye; and
(g) from 0 up to 4% by weight of foam booster,

wherein the polyvinyl alcohol or partially hydrolyzed polyvinyl acetate is extruded.

The toilet rim blocks fabricated from such compositions of matter are those where the preferred maximum mole ratio of acetyl moiety:hydroxyl moiety is about 3:1. It should be noted that partially hydrolyzed polyvinyl acetate having a mole ratio of acetyl moiety:hydroxyl moiety in the range of from 3:1 up to 1:3 is useful in toilet rim blocks, but the majority of the toilet rim block must be fabricated from either extruded polyvinyl alcohol or partially hydrolyzed polyvinyl acetate with the maximum mole ratio of acetyl moiety:hydroxyl moiety being about 3:1. Partially hydrolyzed polyvinyl acetate with a range of mole ratios of acetyl moiety:hydroxyl moiety between about 3:1 and about 1:3 are water-swellable; not water-soluble whereas partially hydrolyzed polyvinyl acetate with a maximum mole ratio of acetyl moiety:hydroxyl moiety of about 1:3 are water-soluble at ambient temperatures, e.g., 25-35°C.

The invention also contemplates a process for preparing the aforementioned composition of matter as well as the toilet rim blocks.

In practicing the invention, the polyvinyl alcohol or partially hydrolyzed polyvinyl acetate is prepared by means of any of Examples I-XIV of U.S. Letters Patent 5,051,222 issued on September 24, 1991. Thus, the polyvinyl alcohol or the partially hydrolyzed polyvinyl acetate is prepared first by polymerizing (via a "free radical" polymerization mechanism) vinyl acetate having the formula:

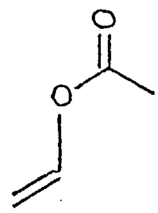

according to the reaction:

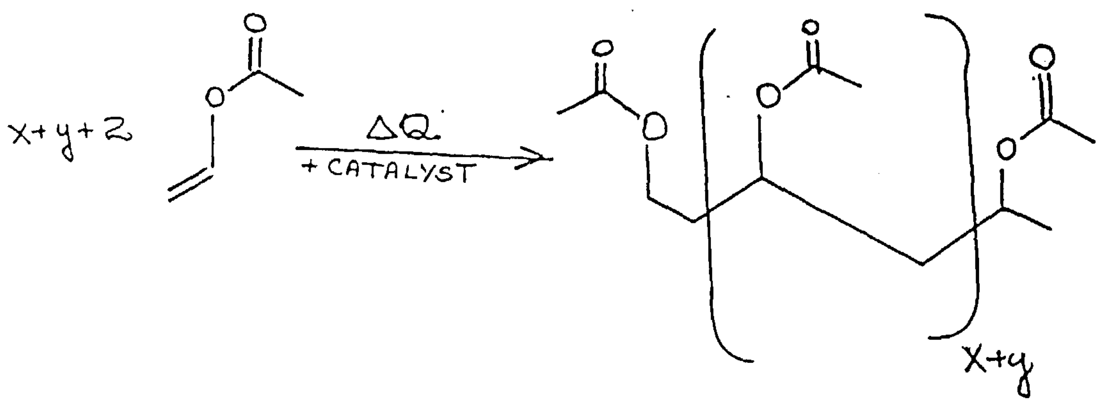


thereby forming a polyvinyl acetate wherein x + y are such that the number average molecular weight of the final product is between 15,000 and 68,000. The resulting polyvinyl acetate having the formula:

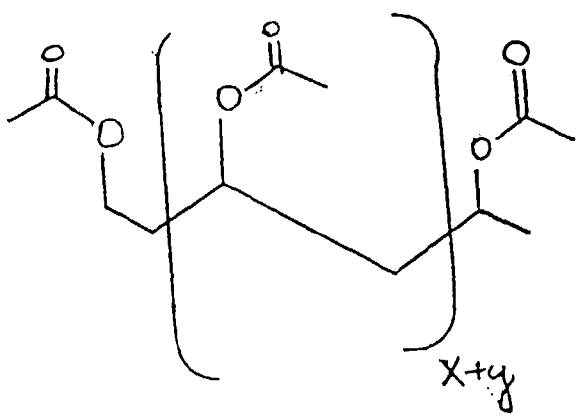

is then hydrolyzed first to form a partially hydrolyzed polyvinyl alcohol according to the reaction:

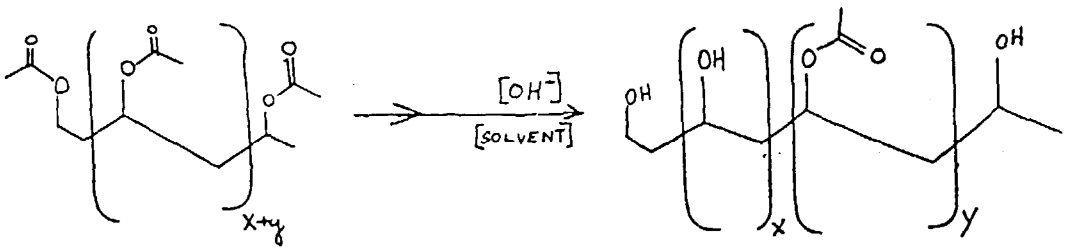


or a mixture of polyvinyl alcohol and partially hydrolyzed polyvinyl acetate according to the reaction:

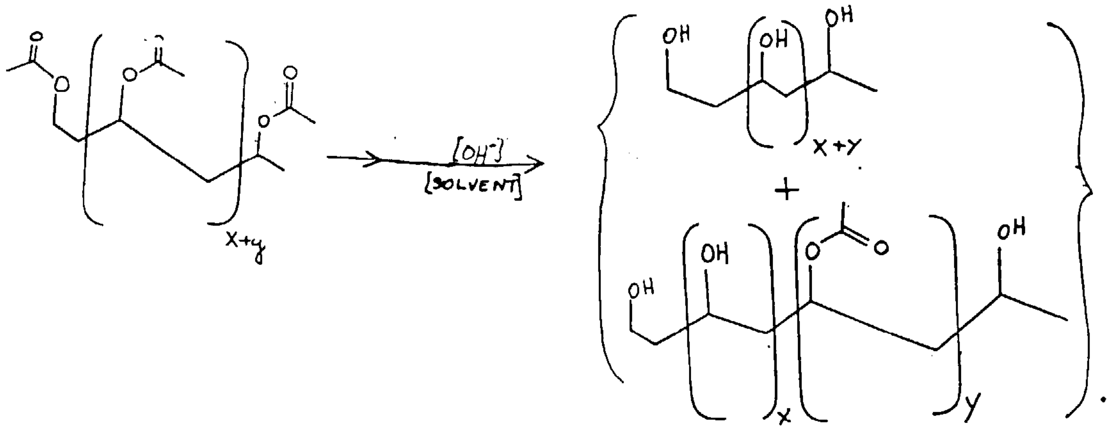


If desired, the partially hydrolyzed polyvinyl acetate may be further hydrolyzed to form polyvinyl alcohol without any acetyl groups present according to the reaction:

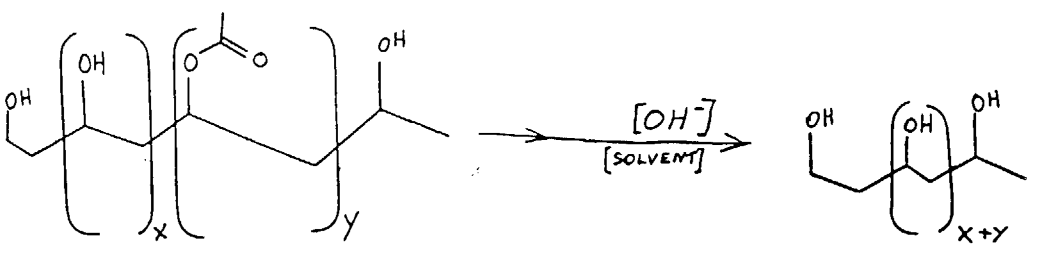


In any event, the ratio of acetyl moieties to hydroxyl moieties is less than about 3:1 in the structure:

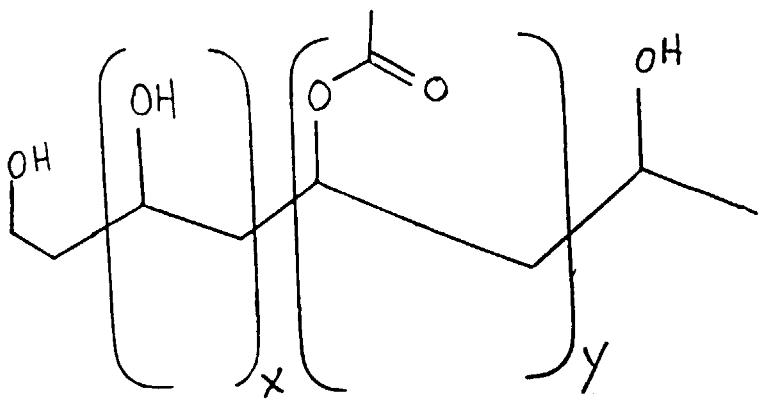

and x and y are defined whereby x + y gives rise to a polymer that has a number average molecular weight of between about 15,000 and 68,000.

The amount of plasticizer contained in the resulting polyvinyl alcohol or partially hydrolyzed polyvinyl acetate may vary from about 0 up to 13% by weight of the polyvinyl alcohol or partially hydrolyzed polyvinyl acetate. This presupposes that the polyvinyl alcohol or partially hydrolyzed polyvinyl acetate commercially available already contains approximately 2% by weight plasticizer. Thus, total plasticizer range varies from about 2% up to about 15%.

The plasticizer is a polar plasticizer having a solubility parameter, $\delta_t$ greater than about 20 **MPa**$^{1/2}$ (megapascals$^{1/2}$) (the square root of cohesive energy density) on the Hildebrand parameter scale (Reference: CRC Handbook of Solubility Parameters and Other Cohesion Parameters; Allen F.M. Barton; published 1983 by the CRC Press, Inc. of Boca Raton, Florida). The solubility parameter, $\delta_t$ is in fact the root mean square sum of the three parameters: $\delta_D$, $\delta_P$, $\delta_H$ according to the equation:

$$\delta_t = \sqrt{\delta_D^2 + \delta_P^2 + \delta_H^2}$$

wherein the parameter $\delta_D$ is a measure of the Van der Waal's forces; $\delta_P$ is a measure of the polar forces and $\delta_H$ is a measure of the hydrogen bonding effect. Examples of such polar plasticizers are:

glycerine;
triethanolamine;
ethylene glycol;
propylene glycol;
diethylene glycol;
dipropylene glycol;
diethanolamine; and
methyldiethylamine.

As stated above from about 1 up to about 20% by weight of a compatible fragrance may be contained within the polyvinyl alcohol or the partially hydrolyzed polyvinyl acetate. The compatible fragrance is added to the extruder at a point downstream from the addition of the polyvinyl alcohol or partially hydrolyzed polyvinyl acetate which is premixed with plasticizer or already contains plasticizer therein.

When from about 1% up to about 10% by weight of the composition of compatible fragrance is added to the extruder, it is not necessary to add to the compatible fragrance hydrophobic silica. When the amount of compatible fragrance is from about 5 up to about 10% by weight of the composition, it is preferred, however, to add hydrophobic silica to the compatible fragrance prior to feeding the mixture thereof to the extruder. When the amount of compatible fragrance desired to be in the polyvinyl alcohol or partially hydrolyzed polyvinyl acetate is from 10% up to about 20% by weight of the composition of compatible fragrance, then although in most cases, it is necessary to add hydrophobic silica to the perfume composition in order to obtain a useful toilet rim block, use of certain polyvinyl alcohol materials such as GOHSENOL® KZ-06 described in detail, infra, have been surprisingly found not to require the use of silica (reference is made to Example III, infra). However, when silica is needed, the weight ratio of hydrophobic silica:compatible fragrance when the compatible fragrance is present in an amount of from about 10 up to about 20% is from about 1:1 silica:compatible fragrance up to about 1:2 silica:compatible fragrance.

An example of hydrophobic silica useful in the practice of the invention is SIPERNAT® D17 silica.

The polyvinyl alcohol or the partially hydrolyzed polyvinyl acetate may be premixed with other additives in addition to the plasticizers, for example, color additives, for example, Acid Blue No. 19 manufactured by Sandoz Pharmaceutical Corporation of Hanover, New Jersey; or antibacterial agents or both.

Examples of the polyvinyl alcohol and partially hydrolyzed polyvinyl acetate useful in the practice of the invention are:

| | |
|---|---|
| AIRVOL® 205 (manufactured by Air Products & Chemicals, Inc. of Allentown, Pennsylvania) | Low Viscosity unplasticized polyvinyl alcohol<br>Molecular Weight: 15,000-27,000<br>Melting Point 230°C<br>88% Hydrolyzed |
| VINEX® 2019 (manufactured by Air Products & Chemicals, Inc. of Allentown, Pennsylvania) | Low Viscosity plasticized polyvinyl alcohol<br>Molecular Weight: 15,000-27,000<br>Melting Point 170°C<br>88% Hydrolyzed |
| VINEX® 2144 (manufactured by Air Products & Chemicals, Inc. of Allentown, Pennsylvania) | Medium Viscosity plasticized polyvinyl alcohol<br>Molecular Weight: 44,000-65,000<br>Melting Point 205°C<br>88% Hydrolyzed |
| VINEX® 1025 (manufactured by Air Products & Chemicals, Inc. of Allentown, Pennsylvania) | Low Viscosity plasticized polyvinyl alcohol<br>Molecular Weight: 15,000-27,000<br>Melting Point 170°C<br>99% Hydrolyzed |
| VINEX® 2025 (manufactured by Air Products & Chemicals, Inc. of Allentown, Pennsylvania) | Low Viscosity plasticized polyvinyl alcohol<br>Molecular Weight: 25,000-45,000<br>Melting Point 192°C<br>88% Hydrolyzed |
| GOHSEFIMER® 5407 (manufactured by Nippon Gohsei K.K. of Osaka, Japan) | Molecular Weight: 23,600<br>Melting Point 100°C<br>30-28% Hydrolyzed |
| GOHSEFIMER® LL02 (manufactured by Nippon Gohsei K.K. of Osaka, Japan) | Molecular Weight: 17,700<br>Melting Point 100°C<br>41-51% Hydrolyzed |
| MOWIOL® 1074 (manufactured by American Hoechst Corp. of Parsippany, New Jersey) | Molecular Weight: 15,000-27,000<br>Melting Point 190°C<br>74% Hydrolyzed |

Furthermore, additional polyvinyl alcohol-containing substances including polyvinyl alcohol and partially hydrolyzed polyvinyl acetate useful in the practice of the invention are as follows:

| Name | Viscosity Range (Centipoises) | Percent Hydrolysis |
|---|---|---|
| ELVANOL® 51-05 (manufactured by The Du Pont Company, Polymer Products Department, Wilmington, Delaware 19898) | 5-6 | 87-89 |
| ELVANOL® 52-22 (manufactured by The Du Pont Company, Polymer Products Department, Wilmington, Delaware 19898) | 21-26 | 87-89 |
| ELVANOL® 50-42 (manufactured by The Du Pont Company, Polymer Products Department, Wilmington, Delaware 19898) | 44-50 | 87-89 |

(continued)

| Name | Viscosity Range (Centipoises) | Percent Hydrolysis |
|---|---|---|
| ELVANOL® 85-82 (manufactured by The Du Pont Company, Polymer Products Department, Wilmington, Delaware 19898) | 24-32 | 99.0-99.8 |
| ELVANOL® 75-15 (manufactured by The Du Pont Company, Polymer Products Department, Wilmington, Delaware 19898) | 12-15 | 98.0-99.8 |
| ELVANOL® T-25 (manufactured by The Du Pont Company, Polymer Products Department, Wilmington, Delaware 19898) | 24-32 | 99.0-99.8 |
| ELVANOL® T-66 (manufactured by The Du Pont Company, Polymer Products Department, Wilmington, Delaware 19898) | 12-15 | 99.0-99.8 |
| ELVANOL® 90-50 (manufactured by The Du Pont Company, Polymer Products Department, Wilmington, Delaware 19898) | 12-15 | 99.0-99.8 |
| ALCOTEX® 72.5 (manufactured by the Harlow Chemical Co. Ltd. of Templefields, Harlow, Essex, England CM20 2BH) | 5.6-6.6 | 71.5-73.5 |
| ALCOTEX® 78 (manufactured by the Harlow Chemical Co. Ltd. of Templefields, Harlow, Essex, England CM20 2BH) | 5.5-6.5 | 76.0-79.0 |
| ALCOTEX® B72 (manufactured by the Harlow Chemical Co. Ltd. of Templefields, Harlow, Essex, England CM20 2BH) | 4.8-5.8 | 72.0-75.0 |
| ALCOTEX® F80/40 (manufactured by the Harlow Chemical Co. Ltd. of Templefields, Harlow, Essex, England CM20 2BH) | 38-42 | 78.8-82.1 |
| ALCOTEX® F88/4 (manufactured by the Harlow Chemical Co. Ltd. of Templefields, Harlow, Essex, England CM20 2BH) | 3.5-4.5 | 86.7-88.7 |
| ALCOTEX® F88/26 (manufactured by the Harlow Chemical Co. Ltd. of Templefields, Harlow, Essex, England CM20 2BH) | 24.5-27.5 | 86.7-88.7 |
| ALCOTEX® F88/40 (manufactured by the Harlow Chemical Co. Ltd. of Templefields, Harlow, Essex, England CM20 2BH) | 38.0-42.0 | 86.7-88.7 |
| ALCOTEX® F88/47 (manufactured by the Harlow Chemical Co. Ltd. of Templefields, Harlow, Essex, England CM20 2BH) | 45.0-49.0 | 86.7-88.7 |
| MOWIOL® 10-74 (manufactured by American Hoechst Celanese Corp., Building 5200, 77 Center Drive, P.O. Box 1026, Charlotte, North Carolina 28201-1026) | 8.5-11.5 | 71.6-75.2 |
| MOWIOL® 15-79 (manufactured by American Hoechst Celanese Corp., Building 5200, 77 Center Drive, P.O. Box 1026, Charlotte, North Carolina 28201-1026) | 13-17 | 79.3-83.7 |

(continued)

| Name | Viscosity Range (Centipoises) | Percent Hydrolysis |
|---|---|---|
| MOWIOL® 4-80 (manufactured by American Hoechst Celanese Corp., Building 5200, 77 Center Drive, P.O. Box 1026, Charlotte, North Carolina 28201-1026) | 3.5-4.5 | 78.8-82.0 |
| MOWIOL® 3-83 (manufactured by American Hoechst Celanese Corp., Building 5200, 77 Center Drive, P.O. Box 1026, Charlotte, North Carolina 28201-1026) | 2.5-3.5 | 80.4-84.8 |
| MOWIOL® 18-88 (manufactured by American Hoechst Celanese Corp., Building 5200, 77 Center Drive, P.O. Box 1026, Charlotte, North Carolina 28201-1026) | 16.5-19.5 | 86.7-88.7 |
| MOWIOL® 40-88 (manufactured by American Hoechst Celanese Corp., Building 5200, 77 Center Drive, P.O. Box 1026, Charlotte, North Carolina 28201-1026) | 38-42 | 86.7-88.7 |
| MOWIOL® 3-98 (manufactured by American Hoechst Celanese Corp., Building 5200, 77 Center Drive, P.O. Box 1026, Charlotte, North Carolina 28201-1026) | 3.0-4.0 | 98.0-98.8 |
| MOWIOL® 6-98 (manufactured by American Hoechst Celanese Corp., Building 5200, 77 Center Drive, P.O. Box 1026, Charlotte, North Carolina 28201-1026) | 5-7 | 98.0-98.8 |
| MOWIOL® 66-100 (manufactured by American Hoechst Celanese Corp., Building 5200, 77 Center Drive, P.O. Box 1026, Charlotte, North Carolina 28201-1026) | 62-70 | 98.8-100.0 |
| GOHSENOL® NK-05 (manufactured by Nippon Gohsei K. K. (The Nippon Synthetic Chemical Industry Co., Ltd.) of No. 9-6, Nozaki-Cho, Kita-Ku, Osaka, 530 Japan) | 4.5-5.5 | 71.0-75.0 |
| GOHSENOL® A-300 (manufactured by Nippon Gohsei K. K. (The Nippon Synthetic Chemical Industry Co., Ltd.) of No. 9-6, Nozaki-Cho, Kita-Ku, Osaka, 530 Japan) | 25-30 | 97-98.5 |
| GOHSENOL® AH-22 (manufactured by Nippon Gohsei K. K. (The Nippon Synthetic Chemical Industry Co., Ltd.) of No. 9-6, Nozaki-Cho, Kita-Ku, Osaka, 530 Japan) | 50-58 | 97.7-98.4 |
| GOHSENOL® C-500 (manufactured by Nippon Gohsei K. K. (The Nippon Synthetic Chemical Industry Co., Ltd.) of No. 9-6, Nozaki-Cho, Kita-Ku, Osaka, 530 Japan) | 25-27 | 96-97 |
| GOHSENOL® GH-20 (manufactured by Nippon Gohsei K. K. (The Nippon Synthetic Chemical Industry Co., Ltd.) of No. 9-6, Nozaki-Cho, Kita-Ku, Osaka, 530 Japan) | 40-46 | 87-89 |
| GOHSENOL® GL-03 (manufactured by Nippon Gohsei K. K. (The Nippon Synthetic Chemical Industry Co., Ltd.) of No. 9-6, Nozaki-Cho, Kita-Ku, Osaka, 530 Japan) | 3.0-3.7 | 87-89 |
| GOHSENOL® GM-14L (manufactured by Nippon Gohsei K.K. (The Nippon Synthetic Chemical Industry Co., Ltd.) of No. 9-6, Nozaki-Cho, Kita-Ku, Osaka, 530 Japan) | 16-20 | 87-89 |
| GOHSENOL® KA-20 (manufactured by Nippon Gohsei K. K. (The Nippon Synthetic Chemical Industry Co., Ltd.) of No. 9-6, Nozaki-Cho, Kita-Ku, Osaka, 530 Japan) | 5-7 | 78-82 |

(continued)

| Name | Viscosity Range (Centipoises) | Percent Hydrolysis |
|---|---|---|
| GOHSENOL® KA-500 (manufactured by Nippon Gohsei K. K. (The Nippon Synthetic Chemical Industry Co., Ltd.) of No. 9-6, Nozaki-Cho, Kita-Ku, Osaka, 530 Japan) | 4.5-7.5 | 70-74 |
| GOHSENOL® KH-20 (manufactured by Nippon Gohsei K. K. (The Nippon Synthetic Chemical Industry Co., Ltd.) of No. 9-6, Nozaki-Cho, Kita-Ku, Osaka, 530 Japan) | 44-52 | 78.5-81.5 |
| GOHSENOL® KP-06 (manufactured by Nippon Gohsei K. K. (The Nippon Synthetic Chemical Industry Co., Ltd.) of No. 9-6, Nozaki-Cho, Kita-Ku, Osaka, 530 Japan) | 5-7 | 71-75 |
| GOHSENOL® N-300 (manufactured by Nippon Gohsei K. K. (The Nippon Synthetic Chemical Industry Co., Ltd.) of No. 9-6, Nozaki-Cho, Kita-Ku, Osaka, 530 Japan) | 25-30 | 98-99 |
| GOHSENOL® NH-26 (manufactured by Nippon Gohsei K. K. (The Nippon Synthetic Chemical Industry Co., Ltd.) of No. 9-6, Nozaki-Cho, Kita-Ku, Osaka, 530 Japan) | 60-68 | 99.4-100 |
| GOHSENOL® NM-11Q (manufactured by Nippon Gohsei K.K. (The Nippon Synthetic Chemical Industry Co., Ltd.) of No. 9-6, Nozaki-Cho, Kita-Ku, Osaka, 530 Japan) | 13-16 | 99-100 |
| GOHSENOL® KZ-06 (manufactured by Nippon Gohsei K. K. (The Nippon Synthetic Chemical Industry Co., Ltd.) of No. 9-6, Nozaki-Cho, Kita-Ku, Osaka, 530 Japan); molecular weight = 42,200; melting point = 140-160°C | | 69.0-72.5 |

Since the VINEX® materials are "preplasticized", no additional plasticizer need be added, however, plasticizer can be added up to an amount of about 13% by weight. On the other hand, the AIRVOL® 205 may be combined with additional plasticizer, for example, glycerine or any of the plasticizers set forth, supra. However, AIRVOL® 205 and polyvinyl alcohol materials similar thereto need not be plasticized to be useful for the purposes of the invention.

The following materials are both foaming agents and are also detergents useful in the practice of the invention:

STANDAPOL® 7023 (an anhydrous blend of Cocamide DEA and DEA-Myreth Sulfate detergent);

WITCONATE® AOK (manufactured by Witco Chemical Comapany (solid anionic alpha sulfonate)); and

WITCONATE® 90 FLAKE (manufactured by Witco Chemical Company (solid anionic alpha sulfonate)).

The following materials are useful as secondary detergents but cannot be depended on for their foaming properties:

PLURONIC® F88 (a block copolymer of ethylene oxide and propylene oxide manufactured by BASF);

PLURONIC® F77 (a block copolymer of ethylene oxide and propylene oxide manufactured by BASF); and

WITCONATE® 90.

Additional examples of detergents which are also foaming agents are alkyl benzene sulfonates such as n-octyl benzene sulfonate (sodium salt).

An example of a foam booster (which is not a detergent) is:

INCROMIDE® CA (Cocamide DEA) manufactured by Croda, Inc..

Another example of a foam booster is a mixture of 1:1 Neodol 45-13:INCROMIDE® CA (Neodol 45-13 being a $C_{14}$-$C_{15}$ linear primary alcohol ethoxylate) (manufactured by the Shell Chemical Company).
Another example of a foaming agent that is also a detergent that is preferred for the practice of the invention is sodium lauryl sulfate.
In carrying out the process of the invention, polyvinyl alcohol or partially hydrolyzed polyvinyl alcohol is combined with plasticizer and added to an extruder, compatible fragrance or compatible fragrance combined with hydrophobic silica is simultaneously fed into the extruder downstream from the addition of the polyvinyl alcohol or partially hydrolyzed polyvinyl acetate/plasticizer mix, and liquid detergent is optionally fed into the extruder upstream from the feeding of the compatible fragrance and downstream from the feeding of the polyvinyl alcohol/plasticizer mix or partially hydrolyzed polyvinyl acetate/plasticizer mix. The extruded tow is cooled (without being immersed in water) and then pelletized. The pelletized product may be marketed as is or it may be formed into toilet rim blocks, for example.
In practicing the process of this invention to form a particulate polyvinyl alcohol/compatible fragrance composition or particulate partially hydrolyzed polyvinyl acetate/compatible fragrance composition, single screw or double screw extruders can be utilized. Some of the extruders that can be used are shown at pages 2416-267 and 332-349 of the Modern Plastics Encyclopedia 1982-1983.
More specifically, examples of extruders which are desirable for carrying out the process of the invention include:

1. The Krauss-Maffei twin screw extruder manufactured by the Krauss-Maffei Corporation/Extruder Division, 3629 West 30th Street, Wichita, Kansas 67277;

2. The CRT ("Counter-Rotating Tangential") Twin Screw Extruder manufactured by Welding Engineers, Inc., King of Prussia, Pennsylvania 19406;

3. The Leistritz Twin Screw Dispersion Compounder manufactured by the American Leistritz Extruder Corporation, 198 U.S. Route 206 South, Sommerville, New Jersey 08876;

4. The ZSK Twin Screw Co-Rotating Extruder manufactured by the Werner & Pfeiderer Corporation, 663 East Crescent Avenue, Ramsey, New Jersey 07446;

5. The MPC/V Baker Perkins Twin Screw Extruder manufactured by the Baker Perkins Inc. Chemical Machinery Division, Saginaw, Michigan 48601;

6. The Berstorff twin screw or foam extrusion equipment manufactured by Berstorff Corporation, P.O. Box 240357, 8200-A Arrowridge Boulevard, Charlotte, North Carolina 28224; and

7. The Theysohn TSK Co-Rotating Extruder manufactured by the Theysohn Corporation of 2 Corporate Drive, Suite F, Radford, Virginia 24141.

Generally, in certain preferred embodiments, twin screw extruders such as those set forth above are used to mix the polyvinyl alcohol or partially hydrolyzed polyvinyl acetate with the perfume, silica (if needed), plasticizer, colorant and detergent (if needed) as well as foaming agent (if needed).
Such extruders comprise an inner shaft member to which an outer screw member is affixed coaxially. In a double-screw machine, there are two shafts, each of which drives an outer screw member. The screws are intermeshed so that they subject the material being extruded to high-shear conditions, which contribute to dispersing the silica/perfume within the polyvinyl alcohol or partially hydrolyzed polyvinyl alcohol polymers.
These extruders also comprise an outer barrel member which encloses the screw or screws. Such extruders over their length can be fitted with different screws on the shaft and with different barrels surrounding the screws. Thus, over the length of the extruder various types and amounts of shear action can be used. The variation is carried out along the length of the extruder so that as various ingredients are initially introduced into the interior of the extruder, as the ingredients are initially mixed, and as the components become more highly mixed or dispersed, the mixing and the shear action can be varied to obtain the particle size and amount of various materials to be dispersed entities in the matrix.
After the extrusion, the extrudate is usually cooled. This can be accomplished by suitable means such as belts, blowers and the like. The cooled extrudate comprises a matrix with dispersed entities. This extrudate is then comminuted to provide the finished particles by means known in the art.
In addition to the use of a single extruder, it will be apparent from this description that a series of extruders can be used to form extrudate. Thus, a perfume can be added in a small percentage in a first extruder followed by

additional perfume and silica in a second extruder. Furthermore, detergent and compatible fragrance can be added in a first extruder followed by detergent, silica and perfume in a second extruder with the detergent being added separately from the silica and perfume.

The extrusion and subsequent comminution enable the facile control of the size of the dispersed entities and the overall size of the particulate fragrancing polymers. This, in turn, provides control over the ultimate properties of the particulate fragranced polymers as taught herein.

**EXAMPLE I**

The following "compatible" rose fragrance formulation is used in carrying out Example II below.

| Ingredient | Parts by Weight |
|---|---|
| Geraniol ex Palmarosa Oil | 0.5 |
| Nerol | 2.0 |
| L-Citronellol | 3.1 |
| D-Citronellol | 3.8 |
| Beta Phenyl Ethyl Alcohol | 2.0 |
| o-(2-hydroxy-1-ethyl)-methylbenzene | 4.0 |
| 5-phenyl-3-methyl-1-pentanol | 5.6 |

It should be noted that each of the ingredients of this example is a highly polar primary organic alcohol. Thus, preferably, the "compatible fragrances" of the invention are mixtures of highly polar primary organic alcohols.

**EXAMPLE II**

VINEX® 2144 is fed at 17.24 Kg (38 pounds) per hour into a 30 mm Werner and Pfleiderer twin screw corotating twin screw extruder. The VINEX® 2144 (produced by Air Products & Chemicals, Inc. of Allentown, Pennsylvania) is melted in the first stage of the screws and fragrance oil is then injected at 0.91 Kg (2 pounds) per hour to yield 95% polyvinyl alcohol-5% fragrance. The melt exits the extruder as two strands are dropped onto a 6.1 m (20') long Sandvik water cooled belt to solidify the melt. The strands are then pelletized with a Conair pelletizer. The Summary of the Extrusion Conditions is set forth in the following Table I:

TABLE I

| | |
|---|---|
| RPM | 350 |
| % Torque | 45-60 |
| Rate Kg per hour (pounds per hour) | 18.14 (40) |
| Melt Temperature | 103-89°C (219°F) |
| Set/Actual Barrel 1 Temperature | 87.78/87.78°C (190/190°F) |
| Set/Actual Barrel 2 Temperature | 93.33/93.33°C (200/200°F) |
| Set/Actual Barrel 3 Temperature | 98.89/98.89°C (210/210°F) |
| Set/Actual Barrel 4 Temperature | 98.89/98.33°C (210/209°F) |
| Set/Actual Barrel 5 Temperature | 98.89/98.89°C (210/210°F) |
| Set/Actual Die Temperature | 98.89/98.33°C (210/209°F) |
| Melt Pressure Kg/cm$^2$ (psi) | 22.5 (320) |

These pellets were extruded into 152.4 mm (6") width-by 0.076 mm (0.003") thick cast film with a 19.05 mm (0.75") diameter Haake single screw extruder. Various molded shapes were made with a 40,640 Kg (40 ton) Nissei injection molding machine. 76.2 mm x 127 mm x 3.175 mm (3" x 5" x 0.125") cards were made on the injection molding machine with the following conditions:

TABLE II

| | |
|---|---|
| RPM | 250 |
| Secondary Pressure | 0.039 Kg/cm$^2$ (0.5 600 psi) |
| Shot Size | 4.5 |
| Injection Speed | 21.0 |
| Rotation Speed | 34.0 |
| Nozzle Temperature | 193.33°C (380°F) |
| Front Temperature | 187.78°C (370°F) |
| Middle Temperature | 182.22°C (360°F) |
| Rear Temperature | 176.67°C (350°F) |
| Eject | 1.1 |
| Decompress | 0.2 |
| Cycle Start | 0.5 |
| Injection Time (seconds) | 12 |
| Curing Time (seconds) | 13 |
| Clamp Pressure | 140.6 Kg/cm$^2$ (2,000 psi) |
| Pump Pressure | 140.6 Kg/cm$^2$ (2,000 psi) |

## EXAMPLE III

GOHSENOL® KZ-06 is fed at either 26.3, 30.84 or 33.11 Kg (58, 68 or 73 pounds) per hour into a 30 mm Werner and Pfleiderer twin screw corotating twin screw extruder. The GOHSENOL® KZ-06 (produced by Nippon Gohsei K.K. of Osaka, Japan) is melted in the first stage of the screws and fragrance oil (of Example I) is then injected at 9.07 Kg (20 pounds) per hour simultaneously with the injection of 9.07, 4.54 or 2.27 Kg (20, 10 or 5 pounds) per hour of WITCONATE® 90 and 0.91 Kg (2 pounds) per hour of foam booster. The melt exits the extruder as two strands which are dropped onto a 6.1 m (20') long Sandvik water cooled belt to solidify the melt. The strands are then pelletized with a Conair pelletizer.

The following Table IV sets forth the materials added to the extruder at GOHSENOL® KZ-06 rates of 26.3, 30.84 or 33.11 Kg (58, 68 and 73 pounds) per hour:

TABLE III

| Ingredients | Parts By Weight | | |
|---|---|---|---|
| | Example IIIA | Example IIIB | Example IIIC |
| GOHSENOL® KZ-06 | 58 | 68 | 73 |
| WITCONATE® 90 | 20 | 10 | 5 |
| Fragrance Oil (Example I) | 20 | 20 | 20 |
| Foam Booster (INCROMIDE® CA) | 2 | 2 | 2 |

The rim block formed with 20% surfactant lasts for 300 flushes. The rim block formed with 10% surfactant lasts through 400 flushes. The rim block formed with 5% surfactant lasts with 450 flushes.

The molded rim blocks are made as follows: the pellets are extruded into 152.4 mm (6") width by 0.076 mm (0.003") thick cast film with a 19.05 mm (0.75") diameter Haake single screw extruder. The molded rim blocks are made with a 40,640 Kg (40 ton) Nissei injection molding machine to form blocks having dimensions of 76.2 mm (3") width x 127 mm (5") length x 19.05 mm (0.75") thickness.

In each of the foregoing Examples I, II and III, in addition to the fragrance of Example I, other "compatible" fragrances of a deodorancy type can be used. An example is the Schiff base of helional and methyl anthranilate. "HELIONAL®" is a registered trademark of International Flavors & Fragrances for Piperonyl Propanal.

The features disclosed in the foregoing description, in the following claims and/or in the accompanying drawings may, both separately and in any combination thereof, be material for realising the invention in diverse forms thereof.

## Claims

1. A composition of matter consisting of:

   (a) polyvinyl alcohol or partially hydrolyzed polyvinyl acetate with a maximum mole ratio of acetyl moiety: hydroxyl moiety being 3:1 having a number average molecular weight of from 15,000 up to 68,000 containing from 0 up to 15% by weight of an added polar plasticizer;

   (b) from 1 up to 20% by weight of compatible fragrance contained within the polyvinyl alcohol or partially hydrolyzed polyvinyl acetate;

   (c) from 0 up to 20% by weight of foaming agent contained within the polyvinyl alcohol or partially hydrolyzed polyvinyl acetate;

   (d) from 0 up to 20% by weight of hydrophobic silica contained within the polyvinyl alcohol or partially hydrolyzed polyvinyl acetate;

   (e) from 0 up to 20% by weight of at least one surfactant in addition to the said foaming agent;

   (f) from 0 up to 5% by weight of water-soluble dye; and

   (g) from 0 up to 4% by weight of foam booster,

   wherein the polyvinyl alcohol or partially hydrolyzed polyvinyl acetate is extruded.

2. A toilet rim block molded using the composition of matter of Claim 1.

3. The composition of matter of Claim 1 containing from 1 up to 5% by weight of compatible fragrance and 0% silica.

4. The composition of matter of Claim 1 containing from 1 up to 20% by weight of compatible fragrance and 0% silica.

5. The composition of matter of Claim 1 containing from 5 up to 20% by weight of compatible fragrance and from 2½ up to 20% by weight of hydrophobic silica.

6. The composition of matter of Claim 5 wherein the ratio of hydrophobic silica:compatible fragrance varies from 1: 1 up to 1:2.

7. A toilet rim block molded using the composition of matter of Claim 3.

8. A toilet rim block molded using the composition of matter of Claim 4.

9. A toilet rim block molded using the composition of matter of Claim 5.

10. A toilet rim block molded using the composition of matter of Claim 6.

11. A process for forming a fragrance-containing polyvinyl alcohol or partially hydrolyzed polyvinyl acetate product according to claim 1, which process comprises the step of introducing into an extruder polyvinyl alcohol containing a plasticizer or partially hydrolyzed polyvinyl acetate containing a plasticizer simultaneously with introducing into the extruder a compatible fragrance.

12. The process of Claim 11 further characterized in that the compatible fragrance is combined with hydrophobic silica.

13. The process of Claim 11 further characterized in that in addition to the introduction of the fragrance into the extruder, there is also introduced into the extruder detergent or foaming agent.

14. The process of Claim 12 further characterized in that in addition to the introduction of the fragrance into the extruder, there is also introduced into the extruder detergent or foaming agent.

## Patentansprüche

1. Zusammensetzung bestehend aus

   (a) Polyvinylalkohol oder teilweise hydrolysiertem Polyvinylacetat mit einem maximalen Molverhältnis an Acetyleinheit zu Hydroxyleinheit von 3:1 und mit einem zahlenmittleren Molekulargewicht von 15.000 bis 68.000 sowie enthaltend 0 bis 15 Gew.-% eines hinzugefügten polaren Weichmachers,

   (b) 1 bis 20 Gew.-% eines in dem Polyvinylalkohol oder dem teilweise hydrolysierten Polyvinylacetat enthaltenen, kompatiblen Parfums,

   (c) 0 bis 20 Gew.-% eines in dem Polyvinylalkohol oder teilweise hydrolysierten Polyvinylacetat enthaltenen, schäumenden Mittels,

   (d) 0 bis 20 Gew.-% in dem Polyvinylalkohol oder teilweise hydrolysierten Polyvinylacetat enthaltenen, hydrophoben Silica,

   (e) 0 bis 20 Gew.-% mindestens eines grenzflächenaktiven Mittels zusätzlich zu dem genannten schäumenden Mittel,

   (f) 0 bis 5 Gew.-% eines wasserlöslichen Farbstoffes und

   (g) 0 bis 4 Gew.-% eines Schaumverstärkers,

   wobei der Polyvinylalkohol oder das teilweise hydrolysierte Polyvinylacetat extrudiert ist.

2. WC-Stein, geformt unter Verwendung der Zusammensetzung nach Anspruch 1.

3. Zusammensetzung nach Anspruch 1 enthaltend 1 bis 5 Gew.-% eines kompatiblen Parfums und 0 % Silica.

4. Zusammensetzung nach Anspruch 1 enthaltend 1 bis 20 Gew.-% eines kompatiblen Parfums und 0 % Silica.

5. Zusammensetzung nach Anspruch 1 enthaltend 5 bis 20 Gew.-% eines kompatiblen Parfums und 2 1/2 bis 20 Gew.-% an hydrophobem Silica.

6. Zusammensetzung nach Anspruch 5, bei der das Verhältnis von hydrophobem Silica zu kompatiblem Parfum 1:1 bis 1:2 beträgt.

7. WC-Stein, geformt unter Verwendung der Zusammensetzung nach Anspruch 3.

8. WC-Stein, geformt unter Verwendung der Zusammensetzung nach Anspruch 4.

9. WC-Stein, geformt unter Verwendung der Zusammensetzung nach Anspruch 5.

10. WC-Stein, geformt unter Verwendung der Zusammensetzung nach Anspruch 6.

11. Verfahren zur Herstellung eines parfumhaltigen Polyvinylalkohol-oder teilweise hydrolysierten Polyvinylacetat-Produktes nach Anspruch 1, bei dem Polyvinylalkohol, der einen Weichmacher enthält oder teilweise hydrolysiertes Polyvinylacetat, das einen Weichmacher enthält, gleichzeitig mit einem kompatiblen Parfum in den Extruder eingeführt werden.

12. Verfahren nach Anspruch 11,
    weiterhin dadurch **gekennzeichnet,**
    daß das kompatible Parfum mit hydrophobem Silica kombiniert ist.

13. Verfahren nach Anspruch 11,
weiter dadurch **gekennzeichnet,**
daß nicht nur das Parfum in den Extruder eingeführt wird, sondern auch ein Detergens oder ein schäumendes Mittel in den Extruder eingeführt wird.

14. Verfahren nach Anspruch 12,
weiterhin dadurch **gekenzeichnet,**
daß nicht nur das Parfum in den Extruder eingeführt wird, sondern auch ein Detergens oder schäumendes Mittel in den Extruder eingeführt wird.

## Revendications

1. Composition de matière constituée de :

   (a) un alcool polyvinylique ou un acétate de polyvinyle partiellement hydrolysé, le rapport molaire maximal partie acétyle:partie hydroxyle étant d'environ 3:1, ayant un poids moléculaire moyen en nombre compris entre 15 000 et 68 000 contenant entre 0 et 15 % en poids d'un plastifiant polaire ajouté;

   (b) entre 1 et 20 % en poids d'un parfum compatible contenu dans l'alcool polyvinylique ou l'acétate de polyvinyle partiellement hydrolysé;

   (c) entre 0 et 20 % en poids d'un agent moussant contenu dans l'alcool polyvinylique ou l'acétate de polyvinyle partiellement hydrolysé;

   (d) entre 0 et 20 % en poids de silice hydrophobe contenue dans l'alcool polyvinylique ou l'acétate de polyvinyle partiellement hydrolysé;

   (e) entre 0 et 20 % en poids d'au moins un tensioactif en plus dudit agent moussant;

   (f) entre 0 et 5 % en poids d'un colorant hydrosoluble; et

   (g) entre 0 et 4 % en poids d'un activateur de mousse.

   dans laquelle l'alcool polyvinylique ou l'acétate de polyvinyle partiellement hydrolysé est extrudé.

2. Bloc moulé pour cuvette de toilettes utilisant la composition de matière selon la revendication 1.

3. Composition de matière selon la revendication 1 contenant entre 1 et 5 % en poids de parfum compatible et 0 % de silice.

4. Composition de matière selon la revendication 1 contenant entre 1 et 20 % en poids de parfum compatible et 0 % de silice.

5. Composition de matière selon la revendication 1 contenant entre 5 et 20 % en poids de parfum compatible et entre 2,5 et 20 % en poids de silice hydrophobe.

6. Composition de matière selon la revendication 5 dans laquelle le rapport silice hydrophobe:parfum compatible varie entre 1:1 et 1:2.

7. Bloc moulé pour cuvette de toilettes utilisant la composition de matière selon la revendication 3.

8. Bloc moulé pour cuvette de toilettes utilisant la composition de matière selon la revendication 4.

9. Bloc moulé pour cuvette de toilettes utilisant la composition de matière selon la revendication 5.

10. Bloc moulé pour cuvette de toilettes utilisant la composition de matière selon la revendication 6.

11. Procédé de formation d'un produit d'alcool polyvinylique ou d'acétate de polyvinyle partiellement hydrolysé contenant du parfum selon la revendication 1, lequel procédé comprend l'étape d'introduction dans une extrudeuse d'alcool polyvinylique contenant un plastifiant ou d'acétate de polyvinyle partiellement hydrolysé contenant un plastifiant simultanément avec l'introduction dans l'extrudeuse d'un parfum compatible.

12. Procédé selon la revendication 11 caractérisé en outre en ce que le parfum compatible est associé avec de la silice hydrophobe.

13. Procédé selon la revendication 11 caractérisé en outre en ce que en plus de l'introduction du parfum dans l'extrudeuse, on introduit aussi dans l'extrudeuse du détergent ou de l'agent moussant.

14. Procédé selon la revendication 12 caractérisé en outre en ce que en plus de l'introduction du parfum dans l'extrudeuse, on introduit aussi dans l'extrudeuse du détergent ou de l'agent moussant.